# EUROPÄISCHE PATENTSCHRIFT

(11) **EP 2 760 901 B1**
(45) Veröffentlichungstag und Bekanntmachung des Hinweises auf die Patenterteilung: **05.12.2018**
(21) Anmeldenummer: 12762599.4
(22) Anmeldetag: 25.09.2012
(51) Int. Cl.: C08G 18/10, C08G 18/48, C08J 9/14, A61L 15/26, A61L 15/42

(54) **ALPHA-ALKOXYSILAN-TERMINIERTES PRÄPOLYMER FÜR SCHNELLHÄRTENDE SPRÜHSCHÄUME MIT VERBESSERTER TREIBGASLÖSLICHKEIT**
ALPHA-ALKOXYSILANE-TERMINATED PREPOLYMER FOR QUICK HARDENING SPRAY FOAMS WITH IMPROVED FUEL GAS SOLUBILITY
PRÉPOLYMÈRES ALPHA-ALKOXYSILANE-TERMINÉS POUR MOUSSES À ATOMISER DURCISSANT RAPIDEMENT ET AYANT UNE SOLUBILITÉ DE GAZ PROPULSEUR AMÉLIORÉE

(30) Priorität: 29.09.2011 EP 11183214
(43) Veröffentlichungstag der Anmeldung: 06.08.2014
(73) Patentinhaber: Covestro Deutschland AG, 51373 Leverkusen (DE)
(72) Erfinder: SCHÖNBERGER, Jan, 42781 Haan (DE)
(74) Vertreter: Levpat
(86) Internationale Anmeldenummer: PCT/EP2012/068833
(87) Internationale Veröffentlichungsnummer: WO 2013/045422

(56) Entgegenhaltungen:
- EP-A1- 0 807 649
- EP-A1- 2 014 314
- WO-A1-00/04069
- WO-A1-2004/104078
- B. Poggenklas, H. Sommer, V. Stanjek*, R. Weidner, J. Weis: "Silane-Crosslinking High-Performance Spray Foams"; "V" In: N. Auner, J. Weis: "Organosilicon Chemistry VI: From Molecules to Materials", 21. Mai 2008 (2008-05-21), Wiley InterScience, Online, XP002671999, ISBN: 9783527312146 Seiten 813-817, das ganze Dokument

## Beschreibung

Die vorliegende Erfindung betrifft ein α-Alkoxysilan-terminiertes Präpolymer, das durch Umsetzung wenigstens eines Polyetherpolyols, eines Polyisocyanats und eines α-Alkoxysilans, das wenigstens eine isocyanatreaktive Gruppe aufweist, herstellbar ist. Weitere Gegenstände der Erfindung sind ein Verfahren zur Herstellung eines erfindungsgemäßen α-Alkoxysilan-terminierten Präpolymers, eine Zusammensetzung, ein Mehrkomponentensystem und eine Druckdose enthaltend ein erfindungsgemäßes α-Alkoxysilan-terminiertes Präpolymer sowie ein durch Polymerisation aus einem erfindungsgemäßen α-Alkoxysilan-terminierten Präpolymer erhältlicher Formkörper.

Sprühbare Zusammensetzungen sind aus dem Stand der Technik bekannt. So existieren sprühbare Montageschäume, die zum Ausfüllen von Hohlräumen beispielsweise im Baubereich eingesetzt werden. Sie werden insbesondere zum Auffüllen von Fugen und Hohlräumen zwischen Fensterrahmen und Türzargen und dem umliegenden Mauerwerk verwendet und zeichnen sich durch gute feuchtigkeitsisolierende Eigenschaften sowie gute Wärmedämmeigenschaften aus. Weitere Anwendungsgebiete solcher sprühbaren Zusammensetzungen sind die Verwendung zur Isolierung von Rohrleitungen oder das Ausschäumen von Hohlräumen in technischen Geräten.

Typischerweise handelt es sich bei diesen vorgenannten Montageschäumen um Polyurethanschäume (PU-Schäume). Die diesen Schäumen zugrundeliegenden Zusammensetzungen bestehen aus unvernetzten Präpolymeren, die über eine hohe Anzahl freier Isocyanatgruppen verfügen. Die freien Isocyanatgruppen sind sehr reaktiv und können bereits bei normaler Umgebungstemperatur unter Einfluss von Wasser bzw. Feuchtigkeit miteinander reagieren und ein polymeres Netzwerk aus den Präpolymeren aufbauen. Neben der Luftfeuchtigkeit dienen auch Alkohole mit zwei oder mehr OH-Gruppen, entsprechende Thiole sowie primäre oder sekundäre Amine sowie Mischungen von diesen als mögliche Reaktionspartner für die vorbezeichneten Isocyanate. Besonders häufig werden hierfür Polyole verwendet. Bei der Reaktion mit Polyolen bzw. Wasser entstehen Urethan- bzw. Harnstoffeinheiten, die aufgrund ihrer Fähigkeit zur Ausbildung von Wasserstoffbrückenbindungen teilkristalline Strukturen im ausgehärteten Schaum ausbilden können. Hierdurch wird die Härte der Schäume sowie deren Druck- und Reißfestigkeit erhöht.

Die Zusammensetzungen werden häufig in Druckdosen eingefüllt und mit einem Treibmittel versehen, das das Aufschäumen der Präpolymere beim Ausbringen aus der Druckdose erleichtert. Zudem reagieren die Isocyanatgruppen des Präpolymers mit der Luftfeuchtigkeit, wobei Kohlendioxid abgespalten wird, welches ebenfalls zum Aufschäumen beiträgt. Bei dieser Reaktion werden die beteiligten Isocyanatgruppen zu Aminen umgewandelt, die ihrerseits mit weiteren Isocyanatgruppen unter Ausbildung eines polymeren Netzwerks reagieren können, mit anderen Worten also der Vernetzungsreaktion nicht verloren gehen.

Die Polyurethanzusammensetzungen können als 1K-Schäume oder auch als zweikomponentige (2K) Schäume hergestellt werden. Während die 1K-Schäume nur durch den Einfluss von Luftfeuchtigkeit aushärten, werden bei den 2K-Schäumen ein Polyisocyanat und ein Polyol getrennt voneinander aufbewahrt und erst unmittelbar vor dem Ausbringen miteinander vermischt. Der Mischvorgang geschieht hierbei entweder in dem Druckkörper der Druckdose, deren Inhalt dann zügig vollständig aufgebraucht werden muss, da die Polymerisationsreaktion unabhängig davon stattfindet, ob der Schaum ausgebracht wird oder nicht. Solche Systeme werden daher häufig auch als 1,5K-Schäume bezeichnet.

Eine andere Möglichkeit besteht darin, eine Zweikammerdruckdose zu verwenden, in der die beiden Komponenten erst im Bereich des Auslassventils miteinander vermischt werden. Der Hauptvorteil der 2K-Schäume gegenüber den 1K-Schäumen besteht in der erheblich schnelleren Aushärtungsreaktion, da diese auch in Abwesenheit von Luftfeuchtigkeit stattfindet. Demgegenüber ist die Aushärtegeschwindigkeit bei 1K-Schäumen durch die Luftfeuchtigkeit bedingt sowie auch durch die Diffusionsgeschwindigkeit der Luftfeuchtigkeit in das ausgeschäumte Material.

Die vorgenannten Zusammensetzungen enthalten typischerweise neben den Präpolymerkomponenten noch weitere Hilfsstoffe, wie beispielsweise Schaumstabilisatoren sowie Katalysatoren, die die Vernetzungsreaktion beschleunigen sollen. Für letztere werden in erster Linie titan- oder zinnorganische Verbindungen verwendet, wie beispielsweise Dibutylzinndilaurat.

Die vorbezeichneten Polyurethanschäume besitzen im ausgehärteten Zustand gute mechanische und wärmedämmende Eigenschaften und haften auf den meisten Untergründen sehr gut.

Allerdings können die vorbezeichneten Polyurethanschäume noch monomere Diisocyanate enthalten, was bei einer Verwendung der Schäume zur Behandlung von Wunden unerwünscht ist.

Um das Gefährdungspotential der vorgenannten Sprühschäume zu reduzieren, werden in DE 43 03 848 A1 Präpolymere beschrieben, die keine monomeren Isocyanate beinhalten bzw. allenfalls nur geringe Konzentrationen an diesen Verbindungen aufweisen. Hier besteht jedoch ein gewisses Risiko, dass das Präpolymer noch freie Isocyanatgruppen aufweisen kann, was wiederum für medizinische Anwendungen unerwünscht ist.

Aus den vorgenannten Gründen wurden in den letzten Jahren polymerisierbare schäumbare Zusammensetzungen entwickelt, welche nicht über freie Isocyanatgruppen aushärten. So sind beispielsweise aus der US 6020389 A1 Silikonschäume bekannt, welche Alkoxy-, Acyloxy- oder Oximo-terminierte Silikonpräpolymere enthalten. Diese Verbindungen polymerisieren über eine Kondensationsreaktion von Siloxangruppen. Nachteilig bei diesen Verbindungen ist ihre lange Aushärtezeit, da sie - wie auch die 1K-Polyurethan-Sprühschäume - auf Luftfeuchtigkeit für die Polymerisationsreaktion angewiesen sind. Insbesondere bei dickeren ausgeschäumten Schichten benötigt die vollständige Abreaktion entsprechend viel Zeit. Dies ist nicht nur unkomfortabel, sondern auch insofern problematisch, als dass die durch das Aussprühen gebildete Schaumstruktur zum Teil wieder zusammenfällt, bevor die Porenwände durch die fortschreitende Polymerisationsreaktion eine ausreichende Eigenfestigkeit aufbauen können.

Aus der WO 00/04069 sind Alkoxysilan-terminierte Polyurethan-präpolymere bekannt. Diese Präpolymere besitzen ein herkömmliches Polyurethanrückgrat, auch als "backbone" bezeichnet, welches auf an sich bekannte Weise durch Umsetzung von difunktionellen Isocyanaten mit Polyolen erhalten wird. Durch Einsatz eines Überschusses von polyfunktionellen Isocyanaten wird erreicht, dass die jeweiligen Endgruppen der Präpolymerketten freie Isocyanatgruppen besitzen. Diese Isocyanat-terminierten Präpolymere werden dann in einem weiteren Reaktionsschritt mit einem Aminoalkyltrialkoxysilan zu den gewünschten Alkoxysilan-terminierten Polyurethanpräpolymeren umgesetzt. Insbesondere wird hierzu Aminopropyltrimethoxysilan verwendet. Das hieraus erhaltene Präpolymer trägt Trimethoxysilan-terminierte Endgruppen, die über einen Propylen-Spacer an das Polyurethanrückgrat angekoppelt sind. Aufgrund der Propylengruppe zwischen dem Siliciumatom und dem Polyurethan-Backbone werden solche Silane auch als γ-Silane bezeichnet.

Bei der Aushärtungsreaktion reagieren die γ-Silane unter Einwirkung von Wasser unter Alkoholabspaltung und bilden hierbei Si-O-Si-Netzwerke aus, wodurch das Präpolymer aushärtet. Auch wenn die γ-Silane aus toxikologischer Sicht unbedenklicher als die Isocyanat-terminierten Polyurethan-Präpolymere sind, besitzen sie dennoch den Nachteil, dass die Aushärtungsreaktion vergleichsweise langsam abläuft. Dieser Nachteil kann nur zum Teil dadurch kompensiert werden, dass γ-Silan-basierte Zusammensetzungen große Mengen an Vernetzungskatalysatoren zugesetzt werden, wie beispielsweise das auch für Polyurethan-Präpolymere verwendete Dibutylzinndilaurat. Dies wirkt sich jedoch zum Teil nachteilig auf die Lagerstabilität solcher Zusammensetzungen aus.

Da auch größere Mengen an Vernetzungskatalysator die Reaktionsträgkeit der γ-Silane nicht vollständig kompensieren können, wurde nach reaktiveren Verbindungstypen geforscht. Solche sind beispielsweise aus der WO 02/066532 A1 bekannt. Auch bei den hier beschriebenen Präpolymeren handelt es sich um Silan-terminierte Polyurethan-Präpolymere. Der wesentliche Unterschied zu den zuvor beschriebenen γ-Silanen besteht darin, dass zwischen dem Polyurethan-Backbone und dem Siliciumatom anstelle der Propylen-Gruppe ein Methylen-Spacer eingesetzt ist. Aufgrund dessen werden diese Silane auch als α-Silane bezeichnet. Der kürzere Abstand des Siliciumatoms zu der stark polaren Harnstoffgruppe des Polyurethan-Backbones erhöht die Reaktivität der an dem Siliciumatom befindlichen Alkoxygruppen (a-Effekt), so dass die Hydrolyse der Alkoxysilangruppen und die anschließende Kondensationsreaktion mit erheblich gesteigerter Geschwindigkeit abläuft.

Nachteilig sowohl bei α-Silanen als auch bei γ-Silanen ist jedoch, dass die Herstellung sprühbarer Schäume aus diesen Präpolymeren äußerst schwierig ist. Insbesondere die Bereitstellung eines in Druckdosen abfüllbaren Sprühschaums, der eine lockere Porenstruktur mit großem Porenvolumen erzeugen können soll, ist kaum zu bewerkstelligen. Der Grund hierfür ist, dass bei der Vernetzungsreaktion im Gegensatz zu den Polyurethan-Schäumen bei Einwirkung von Wasser keine gasförmigen Reaktionsprodukte (wie CO₂ beim Polyurethanschaum) entstehen, sondern Alkohole abgespalten werden, wie beispielsweise Methanol oder Ethanol. Diese Verbindungen sind jedoch im Gegensatz zu einem gasförmigen Reaktionsprodukt nicht in der Lage, aufschäumende Wirkungen zu entfalten, so dass ein aus einer Druckdose ausgesprühter Schaum bis zum Aushärten stark in sich zusammenfällt. Diesem Effekt kann auch mit dem Einsatz von Schaumstabilisatoren nur bedingt entgegnet werden.

Mit diesem Problem befasst sich die EP 1 829 908 A1, in der ein 2K-Silanpräpolymer basierendes System vorgeschlagen wird. Hierbei wird in einer ersten Komponente das Silan-Präpolymer, wie beispielsweise ein silanterminiertes-Polyurethan-Präpolymer, Dibutylzinndilaurat als Katalysator sowie größere Mengen an Calciumcarbonat verwendet. Die zweite Komponente besteht aus einer hochkonzentrierten wässrigen Zitronensäurelösung. Zum Ausschäumen dieses 2K-Schaumes werden die beiden Komponenten miteinander vermischt und über einen Sprayapplikator an gewünschter Stelle ausgebracht. Dabei bewirkt das in der zweiten Komponente enthaltene Wasser die Vernetzungsreaktion des Silan-Präpolymers, wobei das Calciumcarbonat unter Einwirkung der hochkonzentrierten Zitronensäurelösung CO₂ freisetzt. Das Kohlendioxid bewirkt - wie von den Polyurethan-Präpolymeren bekannt - ein Aufschäumen der ausgebrachten Präpolymermischung.

Nachteilig bei diesem System ist jedoch, dass die hochkonzentrierte Zitronensäurelösung einen pH-Wert von etwa 1-2 besitzt und aufgrund dessen ätzende oder zumindest reizende Eigenschaften entfaltet. Insbesondere beim Aussprühen aus Druckdosen kann es zur Aerosolbildung kommen, wodurch die Augen, die Haut und insbesondere auch die Atemwege des Anwenders gereizt werden. Zudem schränkt das ätzende bzw. korrosive Potential der Zitronensäure den Anwendungsbereich der Zusammensetzungen erheblich ein. So ist es beispielsweise undenkbar, solche Zusammensetzungen im medizinischen Bereich als sprühbare Wundauflage unmittelbar auf der Haut, insbesondere einer verletzten Hautstelle bzw. einem verletzten Körperteil aufzubringen.

Ein weiteres bekanntes Problem beim Aussprühen von Silan-vernetzenden Schäumen besteht darin, dass es während der Aushärtung des Schaums zu Rissbildungen im Schaumgefüge kommen kann. Die Rissbildung sei der Veröffentlichung "Silane-Crosslinking High-Performance Spray Foams, Barbara Poggenklas, Heinrich Sommer, Volker Stanjek, Richard Weidner, Johann Weis, Organosilicon Chemistry VI: From Molecules to Materials, [European Silicon Days], München, DE, 11-12 September 2003" zufolge auf eine zu schnelle Abdiffusion des Treibgases aus dem noch frischen Silanschaum zurückzuführen. Zur Verringerung der Diffusionsgeschwindigkeit sei ein weniger polares Treibgas, wie Propan/Butan, besser geeignet, doch löse sich dieses nur schlecht in den bekannten Silan-terminierten Präpolymeren. Die unzureichende Treibgaslöslichkeit wirke sich wiederum nachteilig auf das Ausschäumverhalten aus. Um diesem Problem zu entgegnen, wird in dieser Veröffentlichung vorgeschlagen, in 5 bis 10 % der Endgruppen der α-silanterminierten Präpolymere lange Alkylketten, also unpolare Gruppen, einzubauen, um die Löslichkeit des unpolaren Treibmittels im Polymer zu verbessern. Allerdings werden keine konkreten Angaben gemacht, welche Löslichkeiten für unpolares Treibgas durch diese Maßnahme erzielbar sind. Ungeachtet dessen können durch die Einfügung langer Alkylketten unerwünschte Eigenschaftsveränderungen an den silanterminierten Präpolymeren und den daraus erhältlichen Schäumen auftreten, wie beispielsweise eine deutliche Erhöhung der Schaumhärte. Dies ist nicht in allen Anwendungsgebieten erwünscht.

Die WO 04/104078 A1 befasst sich ebenfalls mit dem zuvor beschriebenen Problem der Rissbildung in Silan-vernetzbaren Sprühschäumen und damit verbunden der beschränkten Löslichkeit von unpolaren Treibgasen in α-Silan-terminierten Präpolymeren. Zur Verbesserung der Löslichkeit von unpolaren Treibgasen wird auch hier eine Modifikation von 5 bis 10 % der Endgruppen der α-silanterminierten Präpolymere durch lange Alkylketten vorgeschlagen. Daraus können, wie oben bereits ausgeführt unerwünschte Eigenschaftsveränderungen der Präpolymere und daraus resultierenden Schäume auftreten. Für den Aufbau der α-Silan-terminierten Präpolymere werden kurzkettige Polyetherpolyole einer Molmasse < 450 g/mol eingesetzt. Die Verwendung solcher Polyole führt zu harten Schäumen, die nur für ein stark begrenztes Anwendungsgebiet eingesetzt werden können.

Vor diesem Hintergrund besteht die Aufgabe der vorliegenden Erfindung darin, ein α-Alkoxysilan-terminiertes Präpolymer zur Verfügung zu stellen, aus dem Sprühschäume hergestellt werden können, welche schnell aushärten, eine stark poröse Struktur mit einem hohen Porenvolumen aufweisen sowie generell gute mechanische Eigenschaften besitzen. Ferner soll das Präpolymer beziehungsweise ein aus diesem Präpolymer erhältlicher Sprühschaum ein breiteres Anwendungsgebiet abdecken.

Diese Aufgabe wird durch ein α-Alkoxysilan-terminiertes Präpolymer der eingangs genannten Art gelöst, bei dem das Polyetherpolyol ein Gewichtsmittel von 500 bis 7000 g/Mol aufweist, Propylenoxideinheiten umfasst, und einen Anteil an Ethylenoxideinheiten von 0 bis 50 Gew.-% bezogen auf das Polyetherpolyol aufweist. Ein solches Präpolymer ist insbesondere zur Verwendung in isocyanatfreien schäumbaren Zusammensetzung geeignet, die wiederum speziell für medizinische Anwendungen wie schäumbare Wundauflagen eingesetzt werden können.

Eine bevorzugte Ausführungsform der Erfindung umfasst ein α-Alkoxysilan-terminiertes Präpolymer der eingangs genannten Art, bei dem das Polyetherpolyol ein Gewichtsmittel von 500 bis 7000 g/mol und Ethylenoxid- und Propylenoxideinheiten aufweist, wobei der Anteil an Ethylenoxideinheiten bis 50 Gew,-% bezogen auf das Polyetherpolyol beträgt.

Erfindungsgemäß ist vorgesehen, dass das α-Alkoxysilan-terminierte Präpolymer α-Silangruppen aufweist. Hierunter wird verstanden, dass die Präpolymere im rechnerischen Mittel wenigstens eine α-Silangruppe pro Präpolymermolekül aufweisen. Es kann jedoch ebenso vorgesehen sein, dass das in der erfindungsgemäßen Zusammensetzung enthaltene Alkoxysilan-terminierte Präpolymer ausschließlich α-Silangruppen aufweist.

Unter einer α-Silangruppe versteht man, wie bereits vorgehend ausgeführt wurde, dass zwischen dem Siliciumatom und dem Polymer-Backbone ein Methylen-Spacer vorhanden ist. Solche Silane zeichnen sich durch eine besondere Reaktionsfreudigkeit in Bezug auf die Kondensationsreaktion aus. Aufgrund dessen ist es im Rahmen der vorliegenden Erfindung möglich, vollständig auf den Einsatz Schwermetall-basierter Vernetzungskatalysatoren wie organische Titanate oder organische Zinn(IV)Verbindungen zu verzichten. Dies ist insbesondere bei medizinischen Anwendungsfeldern für die erfindungsgemäße Zusammensetzung von Vorteil.

Unter isocyanatreaktiven Gruppen werden funktionelle Gruppen verstanden, die unter Wasserstoffabspaltung mit Isocyanatgruppen reagieren können. Bevorzugt handelt es sich bei den isocyanatreaktiven Gruppen um OH-, SH- und / oder Amingruppen.

Überraschenderweise hat sich herausgestellt, dass sich in einem α-Alkoxysilan-terminierten Präpolymer der vorgenannten Art unpolare Treibmittel, wie Alkane oder Alkene, insbesondere technisch bedeutsame Mischungen aus Propan/Butan in hohem Maße lösen. Dadurch ist es möglich, aus diesen Präpolymeren in Druckdosen abfüllbare 1K oder 2K Reaktivschaumzusammensetzungen zur Verfügung zu stellen. Diese lassen sich zu feinporigen und stark porösen Schäumen ausschäumen, wobei die 1K Schaumzusammensetzungen über die Einwirkung von Luftfeuchtigkeit aushärten, während die 2K Schaumzusammensetzungen eine Härterkomponente - im einfachsten Fall ein protisches Lösungsmittel wie Wasser oder ein Alkohol - enthalten und auf diese Weise zur Polymerisation gebracht werden.

Solche 2K Schaumzusammensetzungen können in einer Zweikammer- bzw. Mehrkammerdruckdose abgefüllt und mit Hilfe von Treibgasen ausgeschäumt werden. In einer solchen Druckdose liegen die beiden Komponenten der erfindungsgemäßen Zusammensetzung bis unmittelbar vor dem Ausschäumen getrennt voneinander vor, sodass eine gute Lagerstabilität auch ohne den Zusatz von Wasserfängern oder anderer Stabilisatoren erforderlich wäre. Beim Ausbringen der 2K Zusammensetzung aus der Druckdose erfolgt deren Vermischung zweckmäßigerweise in der Nähe des Auslassventils. Die dabei entstehende Mischung aus erster und zweiter Komponente wird durch das weiterhin darin enthaltene Treibgas nach dem Verlassen der Druckdose unmittelbar aufgeschäumt.

Zur Herstellung des erfindungsgemäßen α-Alkoxysilan-terminierten Präpolymers sind grundsätzlich die dem Fachmann an sich bekannten aromatischen, araliphatischen, aliphatischen oder cycloaliphatischen Polyisocyanate einer NCO-Funktionalität von ≥ 2 geeignet. Beispiele derartiger Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Erfindungsgemäß einsetzbare Polyetherpolyole sind beispielsweise die in der Polyurethanchemie an sich bekannten Polytetramethylenglykolpolyether, die beispielsweise durch Polymerisation von Tetrahydrofuran mittels kationischer Ringöffnung erhältlich sind. Ebenfalls geeignet sind die an sich bekannten Additionsprodukte von Styroloxid, Ethylenoxid, Propylen¬oxid, Butylenoxide und/oder Epichlorhydrin an di- oder polyfunktionelle Startermoleküle. Als Startermoleküle können dabei alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

In weiterer Ausgestaltung des erfindungsgemäßen α-Alkoxysilan-terminierten Präpolymers beträgt der Anteil an Ethylenoxideinheiten höchstens 30 Gew.-% und weiter bevorzugt höchstens 20 Gew.-% bezogen auf das Polyetherpolyol. In besonders bevorzugter Weise beträgt der Anteil an Ethylenoxideinheiten 3 bis 30 Gew.-%, insbesondere 5 bis 20 Gew.-%, jeweils bezogen auf das Polyetherpolyol. Die übrigen Alkylenoxideinheiten können weitestgehend Propylenoxideinheiten sein, es können aber auch andere Struktureinheiten vorhanden sein. Präpolymere mit einer solchen Struktur zeichnen sich durch eine besonders gute Treibgaslöslichkeit von Alkanen bei gleichzeitig guter Flexibilität der erhaltenen Schäume aus.

Es ist jedoch zweckmäßig, den Anteil an Ethylenoxideinheiten im Polyether nicht zu hoch einzustellen, da dies ansonsten zu einem starken Aufquellen der Wundauflage führen würde. Zudem reduziert sich mit steigendem Ethylenoxidanteil die Treibgaslöslichkeit. Insofern ist eine bevorzugte Ausführungsform des erfindungsgemäßen α-Alkoxysilan-terminierten Präpolymers durch einen Anteil an Ethylenoxid-Bausteinen in den vorgenannten Bereichen definiert. Die Untergrenze an Ethylenoxid-Gruppen kann beispielsweise bei etwa 5 Gew.-% liegen. Ungeachtet dessen können auch Polyether ohne Ethylenoxideinheiten verwendet werden.

Nach einer weiteren Ausführungsform des erfindungsgemäßen α-Alkoxysilan-terminierten Präpolymers beträgt das Gewichtsmittel des Polyetherpolyols 800 bis 6000 g/Mol, insbesondere 1000 bis 4500 g/Mol. Solche Präpolymere zeichnen sich ebenfalls durch eine gute Treibgaslöslichkeit in Bezug auf die genannten Alkane aus und ermöglichen gleichzeitig eine flexible Anpassung der Schaumhärte an die gewünschten Anforderungen. Hierbei liefern kurzkettigere Präpolymere in der Regel härtere Schäume als langkettige Präpolymere.

So ist es insbesondere für die vorgenannten medizinischen Anwendungen von Vorteil, dass die Härte des erhaltenen Polymerschaums durch die Wahl der chemischen Natur und/oder der Kettenlänge des Polymerrückgrats des α-Alkoxysilan-terminierten Präpolymers variabel gestaltet werden kann. Neben den vorgenannten Parametern lässt sich die Härte des Schaumes auch durch weitere Maßnahmen, wie beispielsweise durch den Vernetzungsgrad modifizieren. Es können somit sehr weiche und damit nachgebende Polymerschäume oder auch feste Polymerschäume mit stützenden Eigenschaften gebildet werden. Insofern ist der medizinische Anwendungsbereich nicht nur auf die unmittelbare Wundbehandlung beschränkt, sondern es ist auch die Ruhigstellung von Gliedmaßen, beispielsweise bei Knochenbrüchen, Bänderdehnungen, Verstauchungen und dergleichen möglich. Zudem sind ebenfalls Anwendungen im kosmetischen Bereich denkbar.

Gemäß einer weiteren bevorzugten Ausführungsform der Erfindung ist vorgesehen, dass das α-Alkoxysilan-terminierte Präpolymer durch Umsetzung des α-Alkoxysilans mit einem NCO-terminierten Polyurethanpräpolymer herstellbar ist, wobei das NCO-terminierte Polyurethanpräpolymer durch Umsetzung des Polyisocyanats mit dem Polyetherpolyol herstellbar ist. Dabei kann die mittlere NCO-Funktionalität des NCO-terminierten Polyurethanpräpolymers bevorzugt 4 oder weniger und insbesondere 2 bis 4 sein. Ebenfalls bevorzugt ist in diesem Fall, wenn das NCO-terminierte Polyurethanpräpolymer eine dynamische Viskosität bei 23 °C von 200 bis 10000 mPas und insbesondere von 1500 bis 4500 mPas aufweist.

Die Viskosität lässt sich praktischerweise mittels Rotationsviskosimetrie nach DIN 53019 bei 23°C mit einem Rotationsviskosimeter bei einer Rotationsfrequenz von 18 s⁻¹ der Firma Anton Paar Germany GmbH, Ostfildern, DE bestimmen.

Ein α-Alkoxysilan-terminiertes Präpolymer der vorgenannten Art ist insbesondere für medizinische Anwendungen vorteilhaft, weil es eine ausreichend niedrige Viskosität aufweist, so dass es sich leicht ausschäumen lässt.

In weiter bevorzugter Weise handelt es sich bei den α-Silangruppen des erfindungsgemäßen α-Alkoxysilan-terminierten Präpolymers um Triethoxy-α-Silangruppen. Dies ist insofern von Vorteil, als dass bei der Vernetzungsreaktion vergleichsweise ungefährliches Ethanol freigesetzt wird anstelle von Methanol bei den häufig verwendeten Trimethoxy-α-Silanen. Auch wenn die Reaktivität und damit Aushärtegeschwindigkeit der Trimethoxy-α-Silane höher ist als diejenige der Triethoxy-α-Silane, ist die Reaktivität der Triethoxy-α-Silane für die meisten Anwendungen ausreichend hoch. Insbesondere wenn die Zusammensetzung vor der Applikation mit einem protischen Lösungsmittel wie Wasser vermischt wird, härtet das Alkoxysilan-terminierte Präpolymer in der Regel binnen weniger Minuten vollständig aus, teilweise auch nach weniger als einer Minute.

Bevorzugt ist ebenfalls, wenn es sich bei den α-Silangruppen des eingesetzten Alkoxysilan-terminierten Präpolymers um Diethoxy-α-Silangruppen handelt.

Die vorliegende Erfindung betrifft ferner ein Verfahren zur Herstellung eines erfindungsgemäßen α-Alkoxysilan-terminierten Präpolymers umfassend die folgenden Schritte:
- Umsetzung des Polyetherpolyols mit dem Polyisocyanat zu einem NCO-terminierten Polyurethanpräpolymer, und
- Umsetzung des NCO-terminierten Polyurethanpräpolymers mit dem α-Alkoxysilan zum α-Alkoxysilan-terminierten Präpolymer.

Noch ein weiterer Gegenstand der Erfindung ist eine Isocyanatfreie, schäumbare Zusammensetzung, insbesondere für medizinische Anwendungen wie schäumbare Wundauflagen, die ein erfindungsgemäßes α-Alkoxysilan-terminiertes Präpolymer enthält.

Unter isocyanatfrei wird vorliegend ein System verstanden, das weniger als 0,5 Gew.-% isocyanathaltige Komponenten enthält.

Die erfindungsgemäße Zusammensetzung besitzt eine hohe Aushärtegeschwindigkeit. Hierdurch kann die Mischung mehr oder weniger unmittelbar nach dem Ausschäumen bereits eine selbsttragende Schaumstruktur ausbilden, so dass der Schaum bis zur vollständigen Durchhärtung, was in der Regel nur wenige Minuten erfordert, praktisch nicht mehr zusammenfallen kann. Mit anderen Worten stellt die vorliegende Erfindung neben dem 1K System auch ein einsatzfähiges 2K Silanschaumsystem zur Verfügung, aus dem Polymerschäume mit hohem Porenvolumen erhältlich sind, ohne dass es den zusätzlichen Einsatz gasentwickelnder Reaktanden erfordert, wie beispielsweise die Kombination von Calciumcarbonat und Zitronensäure.

Neben der verbesserten Treibgaslöslichkeit besteht ein weiterer Vorteil der erfindungsgemäßen Zusammensetzung darin, dass diese ein aus EO/PO-Einheiten aufgebautes Polymer-Backbone und insofern einen homogenen Aufbau besitzt. So lassen sich aus den erfindungsgemäßen Zusammensetzungen im Vergleich zu den aus dem Stand der Technik bekannten Silanpolymeren mit unpolaren Seitenketten, die im Prinzip den Aufbau eines Block-Co- beziehungsweise Terpolymers besitzen, deutlich flexiblere Schäume herstellen.

Die Isocyanatfreiheit der Zusammensetzung kann durch verschiedene, dem Fachmann an sich bekannte Möglichkeiten erreicht werden. Erfindungsgemäß besonders geeignet ist eine Aufreinigung der Präpolymere über eine Dünnschichtdestillation. Diese Reinigungsprozedur ist besonders vorteilhaft, weil sich herausgestellt hat, dass sich Zusammensetzungen, deren Präpolymere über eine Dünnschichtdestillation von Polyisocyanaten befreit wurden, besser ausschäumen lassen, da sich die Viskositäten der Zusammensetzungen einfacher einstellen lassen und insgesamt weniger viskose Präpolymere erhalten werden. Die Dünnschichtdestillation kann beispielsweise bei Präpolymeren mit einem Polyether-Backbone und daran angekoppelten Polyisocyanat-Einheiten nach der Umsetzung des Polyetherpolyols mit dem Polyisocyanat erfolgen, also vor der Ankopplung der Silangruppe(n) an dieses Zwischenprodukt.

Die erfindungsgemäße Zusammensetzung ist für eine Vielzahl von Applikationen einsetzbar. So eignen sie sich beispielsweise für sämtliche Anwendungsgebiete, in denen die im Stand der Technik bekannten, vorbezeichneten Polyurethanschäume und auch α- bzw. γ-Silanschäume vorgeschlagen werden, also für den Baubereich, zur Isolation von Rohren oder auch zum Ausschäumen von Hohlräumen in Maschinen.

Überraschenderweise hat sich herausgestellt, dass den erfindungsgemäßen Zusammensetzungen neben diesen Einsatzzwecken auch Anwendungsbereiche im medizinischen Sektor eröffnet sind, da kein Einsatz toxischer oder reizender Verbindungen erforderlich ist.

Der medizinische Anwendungsbereich umfasst beispielsweise die Bereitstellung in-situ herstellbarer Wundauflagen. Dazu kann die erfindungsgemäße Zusammensetzung als 1K oder 2K Schaumsystem der vorbezeichneten Art auf Hautverletzungen oder Verletzungen anderer Art aufgesprüht oder anderweitig aufgetragen werden. Dabei haften die ausgeschäumten Zusammensetzungen nicht merklich auf organischem Gewebe wie beispielsweise Wundgewebe und sind zudem aufgrund ihrer Porenstruktur in der Lage, Wundsekrete oder Blut aufzusaugen. Dies ist wohl darin begründet, dass die erfindungsgemäßen Zusammensetzungen beim Aussprühen unter den vorgenannten Bedingungen wenigstens teilweise eine offene Porenstruktur ausbilden und damit saugfähig sind.

Für diesen Anwendungszweck ist auch die Polarität der erfindungsgemäßen Zusammensetzungen und der daraus erhältlichen Schäume von Vorteil, insbesondere im Vergleich zu den aus dem Stand der Technik bekannten Silanpolymeren mit unpolaren Seitenketten. So kann durch die Wahl des Polyethers beziehungsweise der Einstellung seiner Polarität die Hydrophilie des erhaltenen Schaums bedarfsgerecht modifiziert werden, so dass dieser beispielsweise ein besseres Aufnahmevermögen gegenüber wässrigen Flüssigkeiten, wie Blut oder Wundsekreten entfaltet.

Auch wenn die Bereitstellung der erfindungsgemäßen Zusammensetzung in Druckdosen eine komfortable Möglichkeit darstellt, ist die Erfindung jedoch nicht hierauf beschränkt. So lässt sich die erfindungsgemäße Zusammensetzung auch ohne weiteres als Gießmasse verwenden, die entweder an der Luft oder durch vorheriges Vermischen mit einem protischen Lösungsmittel, wie Wasser, aushärtet.

Die erfindungsgemäße Zusammensetzung ist jedoch besonders für die Schaumapplikation aus einer Druckdose geeignet. Deshalb enthält die Zusammensetzung in bevorzugter Weise ein unter Druck verflüssigtes Treibgas. Das Treibgas kann bevorzugt wenigstens ein Alkan oder ein Alken mit jeweils 1 bis 5 Kohlenstoffatomen und insbesondere wenigstens eine Verbindung aus der Gruppe Ethan, Propan, n-Butan, iso-Butan, Pentan sowie Mischungen von diesen umfassen. Es kommen zwar prinzipiell auch andere Treibgase in Frage, wie beispielsweise Dimethylether, jedoch rekrutieren sich die verwendeten Treibgase in besonders bevorzugter Weise aus den vorgenannten Alkanen beziehungsweise Alkenen. Neben der oben genannten guten Löslichkeit dieser Treibgase in der erfindungsgemäßen Zusammensetzung rufen diese zudem im Gegensatz zu Dimethylether bei Kontakt mit offenen Wunden ein weniger brennendes Gefühl beim Patienten hervor.

Die Zusammensetzung kann beispielsweise bei einem Druck von 1,5 bar und bei einer Temperatur von 20°C wenigstens 3 Gew.-% an Treibgasen bezogen auf die Zusammensetzung enthalten wobei das Treibgas vollständig in der Zusammensetzung gelöst ist. Die vollständige Löslichkeit kann bei 20°C in "Schaugläsern zur optischen Prüfung von Aerosolen" der Firma Pamasol Willi Mäder AG, CH ermittelt werden. Die vollständige Löslichkeit im Sinne der vorliegenden Erfindung ist dann gegeben, wenn das Treibgas dauerhaft (> 1h) keine optisch wahrnehmbare zweite Phase ausbildet.

Nach einer besonders bevorzugten Ausführungsform beträgt der Gehalt an gelöstem Treibgas 10 bis 30 Gew.-% bezogen auf die Zusammensetzung, bevorzugt 15 bis 30 Gew.-%.

Ein weiterer Gegenstand der vorliegenden Erfindung ist ein isocyanatfreies Mehrkomponentensystem, insbesondere zur Herstellung von Schäumen für medizinische Produkte wie Wundauflagen mit wenigstens zwei getrennten Komponenten, wobei die erste Komponente eine Zusammensetzung nach einem der Ansprüche 10 bis 12 und die zweite Komponente eine protische Verbindung, bevorzugt ein protisches Lösungsmittel, insbesondere eine wässrige Komponente umfasst.

Die zweite Komponente kann im einfachsten Fall eine wässrige Komponente sein oder sogar aus Wasser bestehen. Zweckmäßigerweise enthält die wässrige Komponente jedoch weitere Bestandteile. So ist es insbesondere vorteilhaft, wenn sie einen pH-Wert von etwa pH 4,0 bis 9,5 aufweist. Denn es hat sich überraschenderweise herausgestellt, dass sich ein α-Alkoxysilan-terminiertes Präpolymer mittels einer wässrigen Komponente bei den vorgenannten pH-Werten in kürzester Zeit aushärten lässt, so dass eine solche Zusammensetzung in einer Zweikammer- bzw. Mehrkammerdruckdose abgefüllt und mit Hilfe von Treibgasen zu stabilen und feinporigen Schäumen aufgeschäumt werden kann. Aufgrund des gemäßigten pH-Bereichs der wässrigen Komponente von etwa pH 4,0 bis 9,5 kann die erfindungsgemäße Zusammensetzung auch beispielsweise unmittelbar auf die humane oder tierische Haut aufgebracht werden.

Zur weiteren Verbesserung der Hautverträglichkeit kann die wässrige Komponente vorzugsweise einen pH-Wert von 4,5 bis 8,0, insbesondere 5,0 bis 6,5 aufweisen. In diesem pH-Bereich treten praktisch keinerlei Hautirritationen selbst bei empfindlicher Haut auf. Gleichzeitig härten die Zusammensetzungen nach dem Vermischen von erster und zweiter Komponente mit der vorgenannten hohen Geschwindigkeit aus.

Die vorgenannten pH-Bereiche lassen sich im Prinzip auf jede erdenkliche Weise einstellen. So kann die wässrige Komponente wenigstens eine Säure, eine Base oder ein Puffersystem enthalten, wobei der Zusatz eines Puffersystems bevorzugt ist. So zeigt beispielsweise der Vergleich zweier Zusammensetzungen, von denen die eine in der wässrigen Komponente eine Säure und die andere in der wässrigen Komponente ein Puffersystem bei gleichem pH-Wert umfasst, dass die Zusammensetzungen mit dem Puffersystem die Aushärtung des Silan-terminierten Präpolymers positiv beeinflussen, insbesondere feinporigere Schäume ausbilden.

Als Säuren kommen organische und anorganische Verbindungen in Betracht, welche zumindest zum Teil wasserlöslich sind und dabei den pH-Wert ins Saure verschieben. Dies sind beispielsweise Mineralsäuren wie Chlorwasserstoff-, Bromwasserstoff-, Schwefel-, Salpeter- oder Phosphorsäure, um nur einige zu nennen. Als organische Säuren können beispielsweise Ameisensäure, Essigsäure, verschiedene α-Chloressigsäuren, Milchsäure, Äpfelsäure, Zitronensäure, Weinsäure, Bernsteinsäure und dergleichen verwendet werden. Auch Mischungen der vorgenannten Stoffe können verwendet werden.

Erfindungsgemäß verwendbare Basen können ebenfalls organischen und anorganischen Ursprungs und zumindest zum Teil wasserlöslich sein und dabei den pH-Wert ins Basische verschieben. Dies sind beispielsweise die Alkali- oder Erdalkalihydroxide wie Natrium- oder Kaliumhydroxid, Ammoniak um nur einige zu nennen. Als organische Basen kommen beispielsweise Stickstoff-enthaltende Verbindungen in Frage wie primäre, sekundäre, tertiäre aliphatische oder cycloaliphatische Amine sowie aromatische Amine. Konkret seien Methylamin, Dimethylamin, Trimethylamin, Ethylamin, Methyldiethanolamin (MDEA), Piperidin und Pyridin lediglich beispielhaft aufgeführt. Zudem können ebenfalls Mischungen der vorgenannten Stoffe eingesetzt werden.

Ein erfindungsgemäß verwendetes Puffersystem umfasst in der Regel eine Mischung aus einer schwachen Säure und ihrer konjugierten Base beziehungsweise umgekehrt. Auch Ampholyte können verwendet werden. Die im Rahmen der vorliegenden Erfindung eingesetzten Puffer sind insbesondere ausgewählt aus Acetatpuffer, Phosphatpuffer, Carbonatpuffer, Citratpuffer, Tartratpuffer, Bernsteinsäurepuffer, TRIS, HEPES, HEPPS, MES, Michaelis-Puffer oder Mischungen hieraus. Die vorliegende Erfindung ist jedoch nicht auf die vorgenannten Systeme beschränkt. Im Prinzip lässt sich jedes Puffersystem verwenden, das derart eingestellt werden kann, dass der beanspruchte pH-Bereich einstellbar ist.

In weiterer Ausgestaltung der erfindungsgemäßen Zusammensetzung beträgt die Konzentration des Puffersystems 0,001 bis 2,0 Mol/l, insbesondere 0,01 bis 0,5 Mol/l. Diese Konzentrationen sind besonders bevorzugt, da einerseits eine ausreichende Pufferkapazität zur Verfügung gestellt wird und andererseits ein Auskristallisieren des Puffers aus der wässrigen Komponente bei üblichen Lagerbedingungen nicht auftritt. Dies wäre beispielsweise beim Einsatz in Druckdosen nachteilig, da auskristallisierte Bestandteile die Mischeinrichtung oder die Düse der Druckdose verstopfen könnte.

In weiter bevorzugter Weise beträgt die Pufferkapazität wenigstens 0,01 Mol/l, insbesondere von 0,02 bis 0,1 Mol/l.

Im Rahmen der vorliegenden Erfindung kann es von Vorteil sein, die Viskosität der wässrigen Komponente anzupassen, beispielsweise um ihre Vermischbarkeit in einer Mischeinrichtung einer Zweikammer-Druckdose mit dem Silan-terminierte Präpolymer zu erleichtern. So kann die dynamische Viskosität der wässrigen Komponente bei 23°C 10 bis 4000 mPas betragen, insbesondere 300 bis 1000 mPas. Die Viskosität lässt sich praktischerweise mittels Rotationsviskosimetrie nach DIN 53019 bei 23°C mit einem Rotationsviskosimeter bei einer Rotationsfrequenz von 18 s⁻¹ der Firma Anton Paar Germany GmbH, Ostfildern, DE bestimmen.

Nach einer besonders bevorzugten Ausgestaltung der erfindungsgemäßen Zusammensetzung kann die wässrige Komponente einen Verdicker aufweisen. Mit Hilfe des Verdickers können einerseits die oben genannten Viskositäten eingestellt werden. Ein weiterer Vorteil des Verdickers besteht darin, dass dieser in gewissem Maße stabilisierende Eigenschaften auf den Schaum hat und insofern dazu beitragen kann, die Schaumstruktur bis zur Erreichung der Eigentragfähigkeit aufrecht zu halten.

Zudem hat sich überraschenderweise gezeigt, dass sich durch den Zusatz von Verdickern, insbesondere von Verdickern auf Stärke- oder Cellulosebasis, eine Reihe von handelsüblichen Treibgasen in der wässrigen Phase lösen. Da die Löslichkeit dieser Treibgase in der das Silan-Präpolymer enthaltenden ersten Komponente eher weniger problematisch ist, wird hierdurch in den jeweiligen Kammern der Mehrkammerdruckdose eine Phasenseparation von Treibgas und der ersten bzw. zweiten Komponente verhindert. Insofern befinden sich das Treibgas und die erste Komponente bzw. das Treibgas und die zweite Komponente bis zum Verlassen der Druckdose in weitestgehend homogener Mischung. Nach dem Vermischen der beiden in der Dose getrennt vorgehaltenen ersten und zweiten Komponente in einer Mischdüse der Druckdose führt das in der Mischung gelöste Treibgas nach dem Verlassen der Druckdose zu einer starken Expansion dieser Mischung, so dass ein feinporiger Schaum erhalten wird. Folglich sind besonders vorteilhaft einzusetzende Verdicker ausgewählt aus Stärke, Stärkederivaten, Dextrin-, Polysaccharidderivaten wie Guargummi, Cellulose, Cellulosederivaten, insbesondere Celluloseethern, Celluloseestern, organischen vollsynthetischen Verdickern auf Basis von Polyacrylsäuren, Polyvinylpyrrolidonen, Poly(meth)acrylverbindungen oder Polyurethanen (assoziative Verdicker) sowie anorganischen Verdickern, wie Bentonite oder Kieselsäuren oder Mischungen hiervon. Als konkrete Beispiele seien Methylcellulose oder Carboxymethylcellulose, beispielsweise als Na-Salz, genannt.

Im Rahmen der vorliegenden Erfindung kann weiterhin vorgesehen sein, dass die wässrige Komponente eine Polyurethandispersion umfasst oder daraus besteht. Hierfür kann beispielsweise eine handelsübliche Polyurethandispersion eingesetzt werden, deren Konzentration gewünschtenfalls auch mit zusätzlichem Wasser verringert werden kann und die dann mit Hilfe der oben genannten Möglichkeiten in den genannten pH-Bereich gebracht wird. Die Verwendung einer Polyurethandispersion ist vorteilhaft, weil auf diese Weise die Treibgaslöslichkeit speziell in Bezug auf die oben genannten Alkane und Alkene in der wässrigen Phase gesteigert werden kann.

Ein weiterer Vorteil der vorgenannten pH-Werte besteht in Kombination mit der Polyurethandispersion zudem darin, dass es in diesen Bereichen in der Regel nicht zu einer Koagulation der Polymerpartikel der Polyurethan-Dispersion kommt, mit anderen Worten die Dispersion unter diesen Bedingungen lagerstabil ist. Überraschenderweise hat sich gezeigt, dass durch den Einsatz einer Polyurethandispersion die Löslichkeit handelsüblicher Treibgase in der wässrigen Komponente weiter gesteigert werden kann. Besonders bevorzugt ist deshalb der Einsatz einer Polyurethandispersion und eines Verdickers der vorgenannten Art.

Als Polyurethandispersion können im Prinzip sämtliche am Markt erhältliche Polyurethandispersionen verwendet werden. Jedoch ist es auch hier von Vorteil, Polyurethandispersionen einzusetzen, welche aus aromatenfreien Isocyanaten hergestellt wurden, da diese insbesondere für medizinische Anwendungen unbedenklicher sind. Zudem kann die Polyurethandispersion auch weitere Inhaltsstoffe beinhalten. In besonders bevorzugter Weise enthält die Polyurethandispersion 5 bis 65 Gew.-% Polyurethan, insbesondere 20 bis 60 Gew.-%.

In Weiterbildung der erfindungsgemäßen Zusammensetzung beträgt das Gewichtsmittel des Polyurethans der Polyurethandispersion 10000 bis 1000000 g/Mol, insbesondere 20000 bis 200000 g/Mol, jeweils ermittelt über Gelpermeationschromatographie gegenüber Polystyrol-Standard in Tetrahydrofuran bei 23°C. Polyurethandispersionen mit solchen Molmassen sind besonders vorteilhaft, da diese lagerstabile Polyurethandispersionen darstellen, die zudem beim Abfüllen in Druckdosen eine gute Löslichkeit des Treibgases in der zweiten Komponente bewirken.

Nach einer besonders bevorzugten Ausführungsform der erfindungsgemäßen Zusammensetzung enthält das Mehrkomponentensystem einen medizinischen und/ oder kosmetischen Wirkstoff. Im Falle der zwei- oder mehrkomponentigen Zusammensetzung kann dieser in der ersten und/oder zweiten Komponente vorgesehen sein. Zwischen den beiden Wirkstoffgruppen existiert keine scharfe Abgrenzung, da viele medizinische Wirkstoffe auch kosmetische Wirkungen entfalten.

In diesem Zusammenhang ist es ebenfalls denkbar, den oder die Wirkstoffe in Form einer weiteren, d.h. dritten oder vierten Komponente vorzusehen und erst unmittelbar vor der Applikation der Zusammensetzung mit der ersten und zweiten Komponente zu vermischen. Aufgrund der Erhöhung der Komplexität der Zusammensetzung mit zunehmender Zahl an separaten Komponenten ist dieser Weg jedoch in der Regel nur dann sinnvoll, wenn die eingesetzten Wirkstoffe sowohl mit der ersten als auch mit der zweiten Komponente unverträglich sind.

Die Wirkstoffe können als reiner Wirkstoff oder aber in verkapselter Form vorliegen, um beispielsweise eine zeitlich verzögerte Abgabe zu erzielen.

Als kosmetische Wirkstoffe kommen insbesondere solche Wirkstoffe in Betracht, welche hautpflegende Eigenschaften besitzen, beispielsweise feuchtigkeitsspendende oder hautberuhigende Wirkstoffe.

Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxid-freisetzende Komponente, bevorzugt L-Arginin oder eine L-Arginin-haltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF α; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α-Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das germizid, bakterizid, bakteriostatisch, fungizid, viruzid, virustatisch und/ oder allgemein mikrobiozid wirkt.

Insbesondere sind solche Stoffe geeignet, die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobielle Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Gemäß einer besonders bevorzugten Ausführungsform der erfindungsgemäßen Zusammensetzung ist vorgesehen, dass sie wenigstens einen medizinischen Wirkstoff enthält, der insbesondere ausgewählt ist aus Substanzen, die unter in vivo-Bedingungen Stickstoffmonoxid freisetzen, sowie Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Der Gehalt der Wirkstoffe richtet sich grundsätzlich in erster Linie an der medizinisch erforderlichen Dosis aus sowie auch an der Verträglichkeit mit den übrigen Bestandteilen der erfindungsgemäßen Zusammensetzung.

Ferner können der erfindungsgemäßen Zusammensetzung auch weitere Hilfsstoffe zugesetzt werden. Hierfür kommen beispielsweise Schaumstabilisatoren, Thixotropiermittel, Verdicker, Antioxidantien, Lichtschutzmittel, Emulgatoren, Weichmacher, Pigmente, Füllstoffe, Additive zur Gebindestabilisierung, Biozide, Colösemittel, und/oder Verlaufshilfsmittel in Frage.

Als Schaumstabilisatoren eignen sich beispielsweise Alkylpolyglycoside. Diese sind nach dem Fachmann an sich bekannten Methoden durch Umsetzung von längerkettigen Monoalkoholen mit Mono-, Di- oder Polysacchariden erhältlich (Kirk-Othmer Encyclopedia of Chemical Technology, John Wiley & Sons, Vol. 24, S. 29) . Die längerkettigen Monoalkohole, die gegebenenfalls auch verzweigt sein können, weisen bevorzugt 4 bis 22 C-Atome, bevorzugt 8 bis 18 C-Atome und besonders bevorzugt 10 bis 12 C-Atome in einem Alkylrest auf. Im Einzelnen genannt seien als längerkettige Monoalkohole 1-Butanol, 1-Propanol, 1-Hexanol, 1-Oktanol, 2-Ethylhexanol, 1-Decanol, 1-Undecanol, 1-Dodecanol (Laurylalkohol), 1-Tetradecanol (Myristylalkohol) und 1-Octadecanol (Stearylalkohol). Selbstverständlich können auch Gemische der genannten längerkettigen Monoalkohole eingesetzt werden.

Bevorzugt weisen diese Alkylpolyglycoside von der Glucose abgeleitete Strukturen auf. Besonders bevorzugt werden Alkylpolyglycoside der Formel (I) eingesetzt.

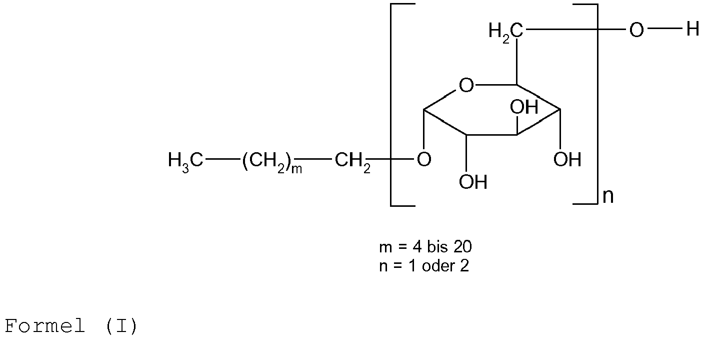

Bevorzugt ist m eine Zahl von 6 bis 20, besonders bevorzugt 10 bis 16.

Die Alkylpolyglycoside weisen bevorzugt einen HLB-Wert von weniger als 20, besonders bevorzugt von weniger als 16 und ganz besonders bevorzugt von weniger als 14 auf, wobei sich der HLB nach der Formel HLB = 20 · Mh/M errechnet, wobei Mh die Molmasse des hydrophilen Anteils eines Moleküls und M die Molmasse des gesamten Moleküls ist (Griffin, W.C.: Classification of surface active agents by HLB, J. Soc. Cosmet. Chem. 1, 1949).

Weitere Schaumstabilisatoren umfassen an sich bekannte anionische, kationische, amphotere und nichtionische Tenside sowie Mischungen hieraus. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt.

Zudem können zur Verbesserung der Schaumeigenschaften des resultierenden Schaums an sich bekannte ein- und mehrwertige Alkohole sowie Mischungen hieraus verwendet werden. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethyl-olpropan, Glycerin, Pentaerythrit, monofunktionelle Polyetheralkohole und Polyesteralkohole, Polyetherdiole und Polyesterdiole.

Diese Schaumstabilisatoren können der ersten und/ oder vorzugsweise der zweiten Komponente zugegeben werden, sofern keine chemische Reaktion mit den jeweiligen Komponenten auftritt. Dabei beträgt der Gesamtgehalt an diesen Verbindungen bezogen auf die erfindungsgemäße Zusammensetzung insbesondere 0,1 bis 20 Gew.-%, bevorzugt 1 bis 10 Gew.-%.

Die Mengenverhältnisse der ersten und zweiten Komponente der erfindungsgemäßen zwei- oder mehrkomponentigen Zusammensetzung werden vorteilhafterweise so zueinander eingestellt, dass eine vollständige Polymerisation erfolgt und dabei die erste Komponente möglichst quantitativ umgesetzt wird. Beispielsweise liegen deshalb die erste und die zweite Komponente der erfindungsgemäßen Zusammensetzung in einem Volumenverhältnis von 1:10 bis 10:1 zueinander vor, vorzugsweise in einem Volumenverhältnis von 1:1 bis 5:1 zueinander, insbesondere 2:1 bis 3:1, besonders bevorzugt bei etwa 2,5:1.

Ein weiterer Gegenstand der vorliegenden Erfindung betrifft einen Formkörper insbesondere in Form einer Wundauflage, der durch vollständige Polymerisation eines erfindungsgemäßen α-Alkoxysilan-terminierten Präpolymers, einer erfindungsgemäßen Zusammensetzung oder eines erfindungsgemäßen Mehrkomponentensystems erhältlich ist. Im erstgenannten Fall erfolgt die Polymerisation beispielsweise unter Einwirkung von Luftfeuchtigkeit. Bei dem Mehrkomponentensystem erfolgt zunächst ein Vermischen der Komponenten und anschließend die vollständige Polymerisation der hierbei entstehenden Mischung. Diese Mischung polymerisiert bevorzugt bei Raumtemperatur in einem Zeitraum von maximal fünf Minuten vollständig, weiter bevorzugt innerhalb von drei Minuten, noch weiter bevorzugt innerhalb von einer Minute. Bei den einkomponentigen Zusammensetzungen hängt die Dauer der vollständigen Polymerisation vornehmlich von der Dicke der ausgebrachten Schicht ab.

Unter vollständiger Polymerisation wird im Sinne der vorliegenden Erfindung verstanden, dass nicht nur eine äußerliche Hautbildung erfolgt, mit anderen Worten, dass die Außenhülle des Formkörpers nicht mehr klebrig ist, sondern dass die Präpolymere weitestgehend vollständig abreagiert sind. Dies wird im Rahmen der vorliegenden Erfindung in der Weise überprüft, dass der hergestellte Formkörper mit einem Finger für einige Sekunden vollständig eingedrückt wird und anschließend bei Wegnahme des Fingerdrucks von selbst in die Ausgangslage zurückkehrt.

Eine derart schnelle Aushärtung ist insbesondere bei medizinischen Anwendungen von Vorteil, speziell bei der Verwendung als sprühbare aufschäumende Wundauflage. Denn erst durch die äußerst schnelle Härtung kann die Wundauflage zeitnah einbandagiert werden und vom Patienten mechanisch belastet werden. Dadurch können lange Wartezeiten vermieden werden.

Ein weiterer Gegenstand der Erfindung ist somit die Verwendung eines insbesondere geschäumten Reaktionsprodukts eines erfindungsgemäßen α-Alkoxysilan-terminierten Präpolymers, einer erfindungsgemäßen Zusammensetzung oder eines erfindungsgemäßen Mehrkomponentensystems als Wundauflage. Eine solche Wundauflage hat den Vorteil, dass durch die Schaumstruktur nicht nur Wundsekrete aufgenommen werden können, sondern hierdurch gleichzeitig ein mechanischer Schutz der Wunde vor Stößen und dergleichen erzielt wird. Auch der Druck von Kleidungsstücken auf der Wunde wird durch die Schaumstruktur teilweise aufgenommen.

Des Weiteren passt sich die gesprühte Wundauflage ideal den zumeist unregelmäßigen Konturen einer Wunde an, gewährleistet somit eine Wundabdeckung weitestgehend frei von Druckschmerzen, welche durch unpassende Wundauflage verursacht werden. Zusätzlich verkürzt die erfindungsgemäß hergestellte Wundauflage den für die Wundversorgung benötigten Zeitaufwand im Vergleich zu einer Versorgung mit einer traditionellen Wundauflage, da keine Anpassung mittels zeitaufwendigen Zuschnitts erforderlich ist.

Die Erfindung betrifft weiterhin auch eine Druckdose, enthaltend ein erfindungsgemäßes α-Alkoxysilan-terminiertes Präpolymer, eine erfindungsgemäße Zusammensetzung oder ein erfindungsgemäßes Mehrkomponentensystem, wobei die Druckdose insbesondere mit einem flüssigen Treibgas mit einem Druck von wenigstens 1,5 bar beaufschlagt ist. Als Treibgase kommen vor allem die bereits oben näher spezifizieren druckverflüssigten Alkane und Alkene in Frage.

In bevorzugter Weise kann zudem in die Druckdose höchstens so viel Treibgas eingefüllt sein, wie der Löslichkeit des Treibgases in der Zusammensetzung beim Fülldruck entspricht. Die Löslichkeit kann über die vorstehend erläuterte fehlende Phasenseparation nach einer Stunde ermittelt werden.

Die Druckdose kann insbesondere als Zwei- oder allgemein als Mehrkammerdruckdose mit einem Auslassventil und einer Mischdüse ausgestaltet sein, wobei die erfindungsgemäße Zusammensetzung in einer ersten Kammer der Zweikammerdruckdose eingefüllt ist und die zweite Kammer eine wässrige Komponente oder ein anderes protisches Lösungsmittel enthält, wobei die erste oder beide Kammern mit einem Flüssigtreibgas unter Überdruck beaufschlagt sind, insbesondere mit einem Druck von wenigstens 1,5 bar. Das Flüssigtreibgas in beiden Kammern kann gleich oder verschieden sein.

Eine für diesen Zweck besonders geeignete Zweikammerdruckdose ist beispielsweise aus den noch nicht veröffentlichten PCT Anmeldungen mit den Anmeldenummern PCT/EP2011/063910 und PCT/EP2011/063909 bekannt, deren Inhalt in die vorliegende Anmeldung vollständig einbezogen wird.

Nach einer weiteren Ausführungsform der erfindungsgemäßen Druckdose sind die Treibgase sowohl in der ersten als auch in der zweiten Komponente löslich, wobei die Löslichkeit bei einem Fülldruck von mindestens 1,5 bar und bei einer Temperatur von 20°C wenigstens 3 Gew.-% beträgt und wobei insbesondere höchstens so viel Treibgas eingefüllt ist, wie der Löslichkeit entspricht. Auf diese Weise wird dafür gesorgt, dass der ausgesprühte Schaum eine gleichbleibende Qualität besitzt, weil nicht aus einer der Kammern zu Beginn des Sprühvorgangs nur Treibgas entweicht und damit das Mischungsverhältnis zwischen erster und zweiter Komponente nicht optimal ist. Hierfür kommen vor allem Zusammensetzungen in Betracht, die in der wässrigen Komponente einen der vorgenannten Verdicker und/ oder eine Polyurethandispersion aufweisen.

Ein weiterer Vorteil ist darin begründet, dass aufgrund der Löslichkeit des Treibgases in den Kammern der Druckdose keine Phasenseparation zwischen erster bzw. zweiter Komponente und dem Treibgas entsteht. Das Treibgas entweicht deshalb erst beim Auslösen der Druckdose und Vermischen der ersten und zweiten Komponente und schäumt diese Mischung dabei auf. Die sehr schnelle Aushärtungszeit der erfindungsgemäßen Zusammensetzung führt dazu, dass die durch das Treibgas aufgeschäumte Schaumstruktur "eingefroren" wird und nicht in sich zusammenfällt.

Der vorgenannte Effekt wird durch den Einsatz eines Verdickers der vorgenannten Art und/ oder einer Polyurethandispersion in der wässrigen Komponente verstärkt, da sowohl der Verdicker als auch die Dispersion in gewissem Maße stabilisierende Eigenschaften auf den Schaum hat. Eine Treibgaslöslichkeit von wenigstens 3 Gew.-% ist von Vorteil, um ein ausreichendes Aufschäumen der ausgebrachten Mischung sicherzustellen. Vorzugsweise enthält die erste Komponente einen Gehalt von 10 bis 40 Gew.-% Treibgas, besonders bevorzugt 15 bis 25 Gew.-% und die zweite Komponente einen Gehalt von 3 bis 20 Gew.-% Treibgas, besonders bevorzugt 5 bis 15 Gew.-% jeweils bezogen auf das resultierende Gesamtgewicht der jeweiligen Mischung. Hierbei kann durch die Menge an eingefüllter bzw. in den einzelnen Komponenten gelöster Treibgasmenge auch die Schaumstruktur beeinflusst werden. So führt bei einer Zusammensetzung eine höhere Treibgasmenge in der Regel zu einem Schaum geringerer Dichte.

Ein weiterer Gegenstand der vorliegenden Erfindung betrifft die Verwendung eines erfindungsgemäßen α-Alkoxysilan-terminierten Präpolymers, einer erfindungsgemäßen Zusammensetzung oder eines erfindungsgemäßen Mehrkomponentensystems zur Herstellung eines geschäumten oder ungeschäumten Polymer-Formkörpers, insbesondere eines flächigen Formkörpers wie einer Wundauflage.

Die vorliegende Erfindung wird im Folgenden anhand von Ausführungsbeispielen näher erläutert:

### Beispiele

### Allgemeines:

Alle folgenden Mengenangaben, Anteile und Prozentanteile sind, soweit nicht anders angegeben, auf das Gewicht und die Gesamtmenge bzw. auf das Gesamtgewicht der Zusammensetzungen bezogen.

Sofern nicht abweichend vermerkt, beziehen sich alle analytischen Messungen auf Messungen bei Temperaturen von 23°C.

### Methoden:

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter bei einer Rotationsfrequenz von 18 s⁻¹ der Firma Anton Paar Germany GmbH, Ostfildern, DE bestimmt.

Die Lagerstabilität der Dispersionen wurde über einen Zeitraum von 6 Monaten nach Herstellung durch Lagerung bei Raumtemperatur überprüft.

Die maximal lösliche Treibgasmenge wurde bei 20°C in "Schaugläsern zur optischen Prüfung von Aerosolen" der Firma Pamasol Willi Mäder AG, CH ermittelt. Die maximal lösliche Treibgasmenge bezieht sich auf das Gewichtsverhältnis von Treibgas zur zu untersuchenden Substanz/Mischung und war erreicht, sobald das Treibgas gerade noch dauerhaft (> 1h) keine zweite Phase ausbildete.

Da eine Viskositätsmessung unter Treibgasbedingungen technisch nicht machbar ist, werden Viskositäten von STP/Treibgas-Lösungen aufgrund der Fließgeschwindigkeit bei 5 % Gefälle in Schaugläsern im Vergleich zu Referenzlösungen mit zuvor bestimmter Viskosität (wässrige Lösungen unterschiedlicher Konzentrationen von Walocel CRT 30 G) abgeschätzt.

Zum Ausschäumen der Mischungen wurde eine 2K-Sprühapparatur verwendet und in der Weise befüllt, wie in den noch nicht veröffentlichten PCT Anmeldungen mit den Anmeldenummern PCT/EP2011/063910 und PCT/EP2011/063909 beschrieben ist.

### Verwendete Substanzen und Abkürzungen:

| | |
|---|---|
| HDI: | Hexamethylen-1,6-diisocyanat |
| Geniosil® XL 926: | [(Cyclohexylamino)methyl] triethoxysilan Wacker Chemie AG, München, DE) |
| Walocel CRT 30G: | Carboxymethylcellulose, Natriumsalz (Dow Deutschland Anlagengesellschaft mbH, Schwalbach, DE) |
| P/B 3,5: | Mischung aus Propan und iso-Butan, so dass bei 20°C ein Gasdruck von 3,5 bar resultiert |

Die folgenden Beispiele zeigen die Herstellung der Silan-terminierten Präpolymere.

### Beispiel 1: Herstellung des Silan-terminierten Präpolymers STP1

Zu einem Gemisch aus 800 g eines Polyalkylenoxids mit einer Molmasse von 2000 g/mol gestartet auf 1,2-Propylenglykol, einem Ethylenoxidgewichtsanteil von 47 % und Propylenoxidgewichtsanteil von 49 %, das zuvor bei 80°C während 1h bei einem Druck von 0,1 mbar getrocknet wurde, und 2,8 g Benzoylchlorid wurde bei 80°C innerhalb von 45 Minuten 1000 g HDI zugetropft und für 2h nachgerührt. Das überschüssige HDI wurde durch Dünnschichtdestillation bei 130°C und 0,4 mbar entfernt. Man erhielt ein Präpolymer mit einem NCO-Gehalt von 3,43 % und einer Viskosität von 1250 mPas.

498 g des erhaltenen Präpolymers wurden anschließend bei 30-40°C innerhalb von 15 Minuten mit 104,5 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Im resultierenden STP konnten 18 % P/B 3,5 lagerstabil gelöst werden.

### Beispiel 2: Herstellung des Silan-terminierten Präpolymers STP2

Zu einem Gemisch aus 1032 g eines Polyalkylenoxids mit einer Molmasse von 4000 g/mol gestartet auf 1,2-Propylenglykol, einem Ethylenoxidgewichtsanteil von 13 % und Propylenoxidgewichtsanteil von 86 %, das zuvor bei 80°C während 1h bei einem Druck von 0,1 mbar getrocknet wurde, und 1,8 g Benzoylchlorid wurde bei 80°C innerhalb von 30 Minuten 650 g HDI zugetropft und für 4h nachgerührt. Das überschüssige HDI wurde durch Dünnschichtdestillation bei 130°C und 0,03 mbar entfernt. Man erhielt ein Präpolymer mit einem NCO-Gehalt von 1,82 % und einer Viskosität von 2100 mPas.

207,5 g des erhaltenen Präpolymers wurden anschließend bei 30-40°C innerhalb von 15 Minuten mit 24,8 g Geniosil XL 926 versetzt. Nach weiteren 30 Minuten Rühren bei 30°C konnte die vollständige Umsetzung des NCO Präpolymers zum STP IR-spektroskopisch nachgewiesen werden. Im resultierenden STP, welches eine Viskosität von 9300 mPas aufwies, konnten 28 % P/B 3,5 lagerstabil gelöst werden. Die Viskosität dieser Lösung wurde bei 26°C auf 400 mPas vergleichend abgeschätzt.

### Beispiel 3: Herstellung des Silan-terminierten Präpolymers STP3

Zu einem Gemisch aus 201 g eines Polyalkylenoxids mit einer Molmasse von 1000 g/mol gestartet auf 1,2-Propylenglykol, einem Ethylenoxidgewichtsanteil von 0 % und einem Propylenoxidgewichtsanteil von 92 %, das zuvor bei 80°C während 1h bei einem Druck von 0,1 mbar getrocknet wurde, und 0,8 g Benzoylchlorid wurde bei 80°C innerhalb von 30 Minuten 588 g HDI zugetropft und für 2h nachgerührt. Das überschüssige HDI wurde durch Dünnschichtdestillation bei 140°C und 0,05 mbar entfernt. Man erhielt ein Präpolymer mit einem NCO-Gehalt von 6,09 %.

200 g des erhaltenen Präpolymers wurden anschließend bei 30-40°C innerhalb von 10 Minuten mit 80 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Im resultierenden STP konnten 22 % P/B 3,5 lagerstabil gelöst werden.

### Beispiel 4: Herstellung des Silan-terminierten Präpolymers STP4

Zu einem Gemisch von 270 g des gemäß Beispiel 1 hergestellten NCO-Präpolymers und 1349 g des gemäß Beispiel 2 hergestellten NCO-Präpolymers wurden bei 30-40°C innerhalb von 30 Minuten 217 g Geniosil XL 926 zugetropft und für weitere 30 Minuten bei 30°C gerührt. Die vollständige Umsetzung des NCO-Präpolymers zum STP konnte IR-spektroskopisch nachgewiesen werden. Im resultierenden STP-Gemisch konnten 41 % P/B 3,5 bzw. 27 % n-Butan lagerstabil gelöst werden.

### Beispiel 5: Herstellung des Silan-terminierten Präpolymers STP5

Zu einem Gemisch aus 423 g eines Polyalkylenoxids mit einer Molmasse von 3825 g/mol gestartet auf Trimethylolpropan, einem Ethylenoxidgewichtsanteil von 13 % und einem Propylenoxidgewichtsanteil von 83 %, das zuvor bei 80°C während 1h bei einem Druck von 0,1 mbar getrocknet wurde, und 0,8 g Benzoylchlorid wurde bei 80°C innerhalb von 30 Minuten 420 g HDI zugetropft und für 2h nachgerührt. Das überschüssige HDI wurde durch Dünnschichtdestillation bei 140°C und 0,05 mbar entfernt. Man erhielt ein Präpolymer mit einem NCO-Gehalt von 2,49 %.

200 g des erhaltenen Präpolymers wurden anschließend bei 30-40°C innerhalb von 10 Minuten mit 37 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Im resultierenden STP konnten 37 % iso-Butan lagerstabil gelöst werden.

### Beispiel 6: Herstellung des Silan-terminierten Präpolymers STP6

Zu einem Gemisch aus 398 g eines Polyalkylenoxids mit einer Molmasse von 4800 g/mol gestartet auf Glycerin, einem Ethylenoxidgewichtsanteil von 13 % und einem Propylenoxidgewichtsanteil von 85 %, das zuvor bei 80°C während 1h bei einem Druck von 0,1 mbar getrocknet wurde, und 0,7 g Benzoylchlorid wurde bei 80°C innerhalb von 30 Minuten 315 g HDI zugetropft und für 2h nachgerührt. Das überschüssige HDI wurde durch Dünnschichtdestillation bei 140°C und 0,05 mbar entfernt. Man erhielt ein Präpolymer mit einem NCO-Gehalt von 1,94 %.

200 g des erhaltenen Präpolymers wurden anschließend bei 30-40°C innerhalb von 10 Minuten mit 28 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Im resultierenden STP konnten 39 % P/B 3,5 bzw. 36 % iso-Butan lagerstabil gelöst werden.

### Vergleichsbeispiel 1: Herstellung eines STP ohne Löslichkeit von Alkan-Treibgasen

Zu einem Gemisch aus 1000 g HDI und 1 g Benzoylchlorid wurde bei 80°C innerhalb von 3h 1000 g eines Polyalkylenoxids mit einer Molmasse von 4680 g/mol gestartet auf Glycerin, einem Ethylenoxidgewichtsanteil von 71 % und Propylenoxidgewichtsanteil von 26 %, das zuvor bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt. Man erhielt ein Präpolymer mit einem NCO-Gehalt von 2,42 % und einer Viskosität von 3500 mPas.

200 g des erhaltenen Präpolymers wurden anschließend bei 30-40°C innerhalb von 10 Minuten mit 31,7 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Im resultierenden STP konnte P/B 3,5 nicht gelöst werden.

In den folgenden Versuchen werden die Ergebnisse der Härtungsversuche der Schäume dargestellt. Das gleichzeitige Ausbringen der beiden Komponenten erfolgte über einen Statikmischer vom Typ MAH 0,5-0,7T der Firma Adchem GmbH,
Wendelstein, DE.

### Beispiel 7: Sprühapplikation

Alle erfindungsgemäßen STP/Treibmittel-Lösungen waren lagerstabil (> 2 Monate) und konnten problemlos aus handelsüblichen Sprühbehältern ausgebracht werden. Hierbei schäumten die Lösungen auf und bildeten einen bis zur vollständigen Aushärtung stabilen Schaum. Auch die Applikation aus einer Pressluft betriebenen 2K Sprühapparatur, wobei die Kammern jeweils getrennt voneinander mit STP/Treibmittel-Lösung in der einen Kammer und mit protischen Flüssigkeiten wie beispielsweise Wasser, wässrigen Säuren, wässrigen Pufferlösungen, wässrigen Katalysatormischungen oder Alkoholen in der zweiten Kammer befüllt wurden und in einem bevorzugten Volumenverhältnis von 2,5 zu 1 zueinander ausgebracht wurden, gelang problemlos und lieferte bis zur vollständigen Aushärtung des jeweiligen STP innerhalb von etwa 5-120 Sekunden stabile Schäume. Es wurden weiche, feinporige Schäume erhalten, welche unter anderem als medizinische Wundauflage geeignet sind.

### Vergleichsbeispiel gemäß EP 1 829 908, Beispiel 1:

Bei diesem Vergleichsversuch soll die erfindungsgemäße Zusammensetzung mit den aus dem Stand der Technik bekannten 2K-Systemen verglichen werden, vorliegend mit dem Beispiel 1 der EP 1 829 908. Bei dem Versuch, die Komponente 2 (8 Teile Wasser, 13 Teile Zitronensäure) mit dem erfindungsgemäß eingesetzten STP 1 über die 2K Sprühapparatur synchron auszubringen, härtete die Mischung bereits im Statikmischer vollständig aus, wodurch dieser blockiert war. Eine Applikation war folglich nicht möglich.

Zudem besitzt die Komponente 2 aufgrund Ihrer Zusammensetzung aus 8 Teilen Wasser und 13 Teilen Zitronensäure einen pH-Wert von etwa 1, wodurch pH-sensible Anwendungen wie beispielsweise medizinische Anwendungen mit diesem System ausgeschlossen sind. Überdies stellt dieser pH-Wert für den Anwender bei der Applikation ein potenzielles Verätzungsrisiko dar.

## Patentansprüche

1. α-Alkoxysilan-terminiertes Präpolymer, das durch Umsetzung wenigstens eines Polyetherpolyols, eines Polyisocyanats und eines α-Alkoxysilans, das wenigstens eine isocyanatreaktive Gruppe aufweist, herstellbar ist, **dadurch gekennzeichnet, dass** das Polyetherpolyol ein Gewichtsmittel von 500 bis 7000 g/Mol aufweist, Propylenoxideinheiten umfasst, und einen Anteil an Ethylenoxideinheiten von 0 bis 50 Gew.-% bezogen auf das Polyetherpolyol aufweist.

2. α-Alkoxysilan-terminiertes Präpolymer nach Anspruch 1, **dadurch gekennzeichnet, dass** der Anteil an Ethylenoxideinheiten höchstens 30 Gew.-% und weiter bevorzugt höchstens 20 Gew.-%. bezogen auf das Polyetherpolyol beträgt.

3. α-Alkoxysilan-terminiertes Präpolymer nach Anspruch 1 oder 2, **dadurch gekennzeichnet, dass** das Gewichtsmittel des Polyetherpolyols 800 bis 6000 g/Mol und weiter bevorzugt 1000 bis 4500 g/Mol beträgt.

4. α-Alkoxysilan-terminiertes Präpolymer nach einem der vorstehenden Ansprüche, **dadurch gekennzeichnet, dass** das α-Alkoxysilan-terminierte Präpolymer Triethoxy-α-Silangruppen enthält.

5. α-Alkoxysilan-terminiertes Präpolymer nach einem der vorstehenden Ansprüche, **dadurch gekennzeichnet, dass** das Polyisocyanat ein aliphatisches Polyisocyanat ist.

6. α-Alkoxysilan-terminiertes Präpolymer nach einem der vorstehenden Ansprüche, **dadurch gekennzeichnet, dass** es durch Umsetzung des α-Alkoxysilans mit einem NCO-terminierten Polyurethanpräpolymer herstellbar ist, wobei das NCO-terminierten Polyurethanpräpolymer durch Umsetzung des Polyisocyanats mit dem Polyetherpolyol herstellbar ist und bevorzugt die mittlere NCO-Funktionalität des NCO-terminierten Polyurethanpräpolymers 4 oder weniger und insbesondere 2 bis 4 ist.

7. Verfahren zur Herstellung eines α-Alkoxysilan-terminierten Präpolymers nach einem der Ansprüche 1 bis 6 umfassend die folgenden Schritte:
• Umsetzung des Polyetherpolyols mit dem Polyisocyanat zu einem NCO-terminierten Polyurethanpräpolymer, und
• Umsetzung des NCO-terminierten Polyurethanpräpolymers mit dem α-Alkoxysilan zum α-Alkoxysilan-terminierten Präpolymer.

8. Isocyanatfreie, schäumbare Zusammensetzung, insbesondere für medizinische Anwendungen wie schäumbare Wundauflagen, **dadurch gekennzeichnet, dass** die Zusammensetzung ein α-Alkoxysilan-terminiertes Präpolymer nach einem der Ansprüche 1 bis 6 enthält.

9. Zusammensetzung nach Anspruch 8, **dadurch gekennzeichnet, dass** sie wenigstens einen medizinischen Wirkstoff enthält, der insbesondere ausgewählt ist aus Substanzen, die unter in vivo-Bedingungen Stickstoffmonoxid freisetzen, sowie Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

10. Zusammensetzung nach einem der Ansprüche 8 oder 9, **dadurch gekennzeichnet, dass** sie ein unter Druck verflüssigtes Treibgas enthält, wobei das Treibgas bevorzugt wenigstens ein Alkan oder ein Alken mit jeweils 1 bis 5 Kohlenstoffatomen und insbesondere wenigstens eine Verbindung aus der Gruppe Ethan, Propan, n-Butan, iso-Butan, Pentan sowie Mischungen von diesen umfasst.

11. Isocyanatfreies Mehrkomponentensystem, insbesondere zur Herstellung von Schäumen für medizinische Produkte wie Wundauflagen mit wenigstens zwei getrennten Komponenten, wobei die erste Komponente eine Zusammensetzung nach einem der Ansprüche 9 bis 10 und die zweite Komponente eine protische Verbindung, bevorzugt ein protisches Lösungsmittel, insbesondere eine wässrige Komponente umfasst.

12. Mehrkomponentensystem nach Anspruch 11, **dadurch gekennzeichnet, dass** die wässrige Komponente einen pH-Wert von pH 4,0 bis 9, 5, bevorzugt von 4, 5 bis 8, 0 und besonders bevorzugt von 5, 0 bis 6,5 aufweist.

13. Mehrkomponentensystem nach einem der Ansprüche 11 oder 12, **dadurch gekennzeichnet, dass** die wässrige Komponente eine Polyurethandispersion umfasst oder daraus besteht.

14. Formkörper, insbesondere in Form einer Wundauflage, erhältlich durch Polymerisation eines α-Alkoxysilan-terminierten Präpolymers nach einem der Ansprüche 1 bis 6, einer Zusammensetzung nach einem der Ansprüche 8 bis 10 oder eines Mehrkomponentensystems nach einem der Ansprüche 11 bis 13.

15. Druckdose, enthaltend ein α-Alkoxysilan-terminiertes Präpolymer nach einem der Ansprüche 1 bis 6, eine Zusammensetzung nach einem der Ansprüche 8 bis 10 oder ein Mehrkomponentensystem nach einem der Ansprüche 11 bis 12, wobei die Druckdose insbesondere mit einem flüssigen Treibgas mit einem Druck von wenigstens 1,5 bar beaufschlagt ist.

## Claims

1. α-Alkoxysilane-terminated prepolymer obtainable by reaction of at least a polyether polyol, a polyisocyanate and an α-alkoxysilane having at least one isocyanate-reactive group, **characterized in that** the polyether polyol has a weight average of 500 to 7000 g/mol, comprises propylene oxide units and has a 0 to 50 wt% fraction, based on the polyether polyol, of ethylene oxide units.

2. α-Alkoxysilane-terminated prepolymer according to Claim 1, **characterized in that** the fraction of ethylene oxide units is not more than 30 wt% and further preferably not more than 20 wt%, based on the polyether polyol.

3. α-Alkoxysilane-terminated prepolymer according to Claim 1 or 2, **characterized in that** the weight average of the polyether polyol is in the range from 800 to 6000 g/mol and further preferably in the range from 1000 to 4500 g/mol.

4. α-Alkoxysilane-terminated prepolymer according to any preceding claim, **characterized in that** the α-alkoxysilane-terminated prepolymer contains triethoxy-α-silane groups.

5. α-Alkoxysilane-terminated prepolymer according to any preceding claim, **characterized in that** the polyisocyanate is an aliphatic polyisocyanate.

6. α-Alkoxysilane-terminated prepolymer according to any preceding claim, **characterized in that** it is obtainable by reacting the α-alkoxysilane with an NCO-terminated polyurethane prepolymer, wherein the NCO-terminated polyurethane prepolymer is obtainable by reacting the polyisocyanate with the polyether polyol and the average NCO functionality of the NCO-terminated polyurethane prepolymer is preferably 4 or less and in particular in the range from 2 to 4.

7. Process for preparing an α-alkoxysilane-terminated prepolymer according to any of Claims 1 to 6, comprising the steps of:
• reacting the polyether polyol with the polyisocyanate to form an NCO-terminated polyurethane prepolymer, and
• reacting the NCO-terminated polyurethane prepolymer with the α-alkoxysilane to form the α-alkoxysilane-terminated prepolymer.

8. Isocyanate-free, foamable composition, in particular for medical applications such as foamable wound dressings, **characterized in that** the composition contains an α-alkoxysilane-terminated prepolymer according to any of Claims 1 to 6.

9. Composition according to Claim 8, **characterized in that** it contains at least an active medical ingredient particularly selected from substances that release nitrogen monoxide under in vivo conditions, and also substance selected from the group of vitamins or provitamins, carotenoids, analgesics, antiseptics, hemostyptics, antihistamines, antimicrobial metals or salts thereof, plant-based wound healing promoter substances or substance mixtures, plant extracts, enzymes, growth factors, enzyme inhibitors and also combinations thereof.

10. Compostion according to either of Claims 8 and 9, **characterized in that** it contains a pressure-liquefied propellant gas, wherein the propellant gas preferably comprises at least one alkane or alkene each of 1 to 5 carbon atoms and more particularly at least one compound from the group ethane, propane, n-butane, isobutane, pentane and also mixtures thereof.

11. Isocyanate-free multicomponent system, in particular for producing foams for medical products such as wound dressings having at least two separate components, wherein the first component comprises a composition according to any of Claims 9 to 10 and the second component comprises a protic compound, preferably a protic solvent, in particular an aqueous component.

12. Multicomponent system according to Claim 11, **characterized in that** the aqueous component has a pH of pH 4.0 to 9.5, preferably of 4.5 to 8.0 and more preferably of 5.0 to 6.5.

13. Multicomponent system according to either of Claims 11 and 12, **characterized in that** the aqueous component comprises or consists of a polyurethane dispersion.

14. Shaped article, in particular in the form of a wound dressing, obtainable by polymerizing an α-alkoxysilane-terminated prepolymer according to any of Claims 1 to 6, a composition according to any of Claims 8 to 10 or a multicomponent system according to any of Claims 11 to 13.

15. Pressurized can containing an α-alkoxysilane-terminated prepolymer according to any of Claims 1 to 6, a composition according to any of Claims 8 to 10 or a multicomponent system according to any of Claims 11 to 12, wherein the pressurized can is more particularly pressurized with a liquid propellant gas to a pressure of at least 1.5 bar.

## Revendications

1. Prépolymère à terminaison α-alcoxysilane, qui peut être fabriqué par mise en réaction d'au moins un polyéther-polyol, un polyisocyanate et un α-alcoxysilane qui comprend au moins un groupe réactif avec les isocyanates, **caractérisé en ce que** le polyéther-polyol présente une moyenne en poids de 500 à 7 000 g/mol, comprend des unités oxyde de propylène et présente une proportion d'unités oxyde d'éthylène de 0 à 50 % en poids, par rapport au polyéther-polyol.

2. Prépolymère à terminaison α-alcoxysilane selon la revendication 1, **caractérisé en ce que** la proportion d'unités oxyde d'éthylène est d'au plus 30 % en poids et de manière davantage préférée d'au plus 20 % en poids, par rapport au polyéther-polyol.

3. Prépolymère à terminaison α-alcoxysilane selon la revendication 1 ou 2, **caractérisé en ce que** la moyenne en poids du polyéther-polyol est de 800 à 6 000 g/mol et de manière davantage préférée de 1 000 à 4 500 g/mol.

4. Prépolymère à terminaison α-alcoxysilane selon l'une quelconque des revendications précédentes, **caractérisé en ce que** le prépolymère à terminaison α-alcoxysilane contient des groupes triéthoxy-α-silane.

5. Prépolymère à terminaison α-alcoxysilane selon l'une quelconque des revendications précédentes, **caractérisé en ce que** le polyisocyanate est un polyisocyanate aliphatique.

6. Prépolymère à terminaison α-alcoxysilane selon l'une quelconque des revendications précédentes, **caractérisé en ce qu'**il peut être fabriqué par mise en réaction de l'α-alcoxysilane avec un prépolymère de polyuréthane à terminaison NCO, le prépolymère de polyuréthane à terminaison NCO pouvant être fabriqué par mise en réaction du polyisocyanate avec le polyéther-polyol, et la fonctionnalisé NCO moyenne du prépolymère de polyuréthane à terminaison NCO étant de préférence de 4 ou moins, et notamment de 2 à 4.

7. Procédé de fabrication d'un prépolymère à terminaison α-alcoxysilane selon l'une quelconque des revendications 1 à 6, comprenant les étapes suivantes :
- la mise en réaction du polyéther-polyol avec le polyisocyanate pour former un prépolymère de polyuréthane à terminaison NCO, et
- la mise en réaction du prépolymère de polyuréthane à terminaison NCO avec l'α-alcoxysilane pour former le prépolymère à terminaison α-alcoxysilane.

8. Composition moussable sans isocyanate, notamment pour des applications médicales telles que des pansements moussables, **caractérisée en ce que** la composition contient un prépolymère à terminaison α-alcoxysilane selon l'une quelconque des revendications 1 à 6.

9. Composition selon la revendication 8, **caractérisée en ce qu'**elle contient au moins un agent actif médical, qui est notamment choisi parmi les substances qui libèrent du monoxyde d'azote en conditions in vivo, ainsi qu'une substance choisie dans le groupe constitué par les vitamines ou les provitamines, les caroténoïdes, les analgésiques, les antiseptiques, les hémostyptiques, les antihistaminiques, les métaux antimicrobiens ou leurs sels, les substances ou mélanges de substances végétales favorisant la cicatrisation, les extraits de plantes, les enzymes, les facteurs de croissance, les inhibiteurs enzymatiques, ainsi que les combinaisons de ceux-ci.

10. Composition selon l'une quelconque des revendications 8 ou 9, **caractérisée en ce qu'**elle contient un gaz propulseur liquéfié sous pression, le gaz propulseur comprenant de préférence au moins un alcane ou un alcène contenant à chaque fois 1 à 5 atomes de carbone et notamment au moins un composé du groupe constitué par l'éthane, le propane, le n-butane, l'isobutane, le pentane, ainsi que les mélanges de ceux-ci.

11. Système multicomposant sans isocyanate, notamment pour la fabrication de mousses pour produits médicaux tels que des pansements, contenant au moins deux composants séparés, le premier composant comprenant une composition selon l'une quelconque des revendications 9 à 10 et le deuxième composant comprenant un composé protique, de préférence un solvant protique, notamment un composant aqueux.

12. Système multicomposant selon la revendication 11, **caractérisé en ce que** le composant aqueux présente un pH de 4,0 à 9,5, de préférence de 4,5 à 8,0 et de manière particulièrement préférée de 5,0 à 6,5.

13. Système multicomposant selon l'une quelconque des revendications 11 ou 12, **caractérisé en ce que** le composant aqueux comprend une dispersion de polyuréthane ou en est constitué.

14. Corps moulé, notamment sous la forme d'un pansement, pouvant être obtenu par polymérisation d'un prépolymère à terminaison α-alcoxysilane selon l'une quelconque des revendications 1 à 6, d'une composition selon l'une quelconque des revendications 8 à 10 ou d'un système multicomposant selon l'une quelconque des revendications 11 à 13.

15. Boîte sous pression, contenant un prépolymère à terminaison α-alcoxysilane selon l'une quelconque des revendications 1 à 6, une composition selon l'une quelconque des revendications 8 à 10 ou un système multicomposant selon l'une quelconque des revendications 11 à 12, la boîte sous pression étant notamment chargée avec un gaz propulseur liquide à une pression d'au moins 1,5 bar.
